# EUROPEAN PATENT APPLICATION

(11) **EP 4 464 766 A1**
(43) Date of publication of application: **20.11.2024**
(21) Application number: 23740347.2
(22) Date of filing: 13.01.2023
(51) Int. Cl.: C12M 1/00, C12N 5/071, C12N 5/077

(54) **CONTAINER FOR PRODUCING TENSION-APPLIED THREE-DIMENSIONAL ARTIFICIAL SKIN AND PRODUCTION METHOD THEREOF**

(30) Priority: 13.01.2022 JP 2022003442
(71) Applicant: RIKEN, Wako-shi, Saitama 351-0198 (JP); Organtech, Inc., Tokyo 104-0028 (JP); JMS Co., Ltd., Hiroshima-shi Hiroshima 730-8652 (JP)
(72) Inventor: TSUJI, Takashi, Wako-shi, Saitama 351-0198 (JP); OGAWA, Miho, Wako-shi, Saitama 351-0198 (JP); HIRAI, Satoshi, Hiroshima-shi, Hiroshima 730-8652 (JP)
(74) Representative: V.O.
(86) International application number: PCT/JP2023/000894
(87) International publication number: WO 2023/136339

(57) **Abstract**

To provide a culture insert container that will improve yield and eliminate various operations requiring proficiency, and dramatically increase production efficiency, and permit reduction in the amount of fibroblast cells that are used, without requiring an operation in which a piece is cut out therefrom, and to provide a method for producing tension-applied three-dimensional artificial skin employing same, a culture insert container having a lower culture container having a bottom portion equipped with a porous membrane and a sidewall, and having an upper insertion fixture having a cylindrical sidewall that is inserted into said lower culture container, said cylindrical sidewall having a land arranged so as to protrude from a top end of the sidewall of the lower culture container at a location such as will cause a top surface of said porous membrane and a bottom end portion of said cylindrical sidewall which is inserted within the lower culture container to be maintained in a noncontacting state, and having a land arranged at an outer surface of the cylindrical sidewall so as to cause a constant distance to be maintained between an inner surface of the sidewall of the lower culture container and the outer surface of the cylindrical sidewall, is employed.

## Description

### TECHNICAL FIELD

The present invention relates to a container for producing tension-applied three-dimensional artificial skin and a production method therefor.

### BACKGROUND ART

With respect to artificial skin, there has until now been development of not only three-dimensional epidermal models but also of Bell-type artificial skin having an epidermis and a dermis. In addition, as attempts have been made to use artificial skin to perform testing to evaluate safety and functionality primarily of materials in which cosmetics have been blended, as well as testing of microstimulation of skin and the like, various artificial skin products are sold so that they might be available for such testing.

However, as the artificial skins that have been sold to date have primarily been predicated on use in testing for evaluation of safety, the focus has been on obtaining consistent and universal results with regard to product lot stability, evaluation of adequacy of functionality, reactivity, and so forth. Moreover, because the artificial skins that have been sold to date have not reflected the physiological environment of the skin of a living organism, it has been hoped that development of higher-level three-dimensional artificial skins might take place.

The inventor(s) therefore proceeded to improve artificial skin with the intention of reproducing the physiological environment of the skin; and in particular, with the goal of making it possible for this to be employed for evaluation of function, selected suitable human epidermal cells and dermal fibroblasts; and also, to reproduce the skin structure, created a three-dimensional artificial skin in which there were two layers of differing dermal cell density; and moreover, utilized a technique for imparting tensile mechanical stress to that three-dimensional artificial skin, as a result of which a tension-applied three-dimensional artificial skin which made it possible for safety testing similar to that performed conventionally to be carried out, and which permitted further improvement in the sensitivity with which functionality could be evaluated as compared with that possible with conventional three-dimensional skin, was developed and proposed (see Nonpatent Reference No. 1).

This tension-applied three-dimensional artificial skin has an epidermis in the form of epidermal layers comprising four layers, these respectively being an epidermal basal cell layer, a prickle cell layer, a granule cell layer, and a cornified layer; and has a dermis comprising two layers, these being a papillary layer and a reticular layer of differing cell density. In addition, inasmuch as it has been acknowledged that, like human skin, there is an extracellular matrix and expression of proteins peculiar to cells, it is fair to say that this tension-applied three-dimensional artificial skin is a model that also reproduces human skin in terms of the extracellular matrix and protein expression thereof. Furthermore, by causing lateral tensile mechanical stresses peculiar to skin to be imparted thereto, the orientation of fibroblasts and the extracellular matrix in this tension-applied three-dimensional artificial skin is made to be more like that of natural skin than is the case with conventional Bell-type artificial skin.

For this reason, not only is it possible with tension-applied three-dimensional artificial skin to carry out conventional safety tests such as cell viability testing, water loss testing, and electrical conductivity testing in similar fashion, but it is also possible to carry out epidermal and dermal gene expression, immunostaining, and so forth for stable evaluation of the functions of various components. Testing in which epidermal layer(s) are coated to reproduce testing of coatings on the skin, testing in which various components are added to culture medium to reproduce the blood-borne route, and so forth are employed as such testing methods, all of which are characterized by the fact that broad evaluation of the functions of the various components which are the targets of testing is permitted thereby.

It is thus thought that imparting tensile mechanical stress thereto causes the tension-applied three-dimensional artificial skin to be a model that reproduces the tissue structure and physiological function of the skin. Tension-applied three-dimensional artificial skin is therefore-with respect to the evaluation of functionality that is permitted thereby-a functionality evaluation system which permits evaluation of increase or decrease in various genes or proteins, and is also capable of being applied to skin injury models such as those involving response to oxidative stress and so forth, and of being applied to skin microstimulation and hypersensitivity models.

### PRIOR ART REFERENCES

### PATENT REFERENCES

Nonpatent Reference No. 1: Communications Biology 3, 637, 2020

### SUMMARY OF INVENTION

### PROBLEM TO BE SOLVED BY INVENTION

The tension-applied three-dimensional artificial skin proposed by the present inventor(s) at Nonpatent Reference No. 1 reproduces a skin tissue structure and physiological function that are similar to those of natural skin. The conventional method for producing this tension-applied three-dimensional artificial skin has been to first form a fibroblast layer in a Petri dish and thereafter cut out a piece therefrom which is of a size corresponding to a Snapwell insert, and to then cause the piece of the fibroblast layer that was cut out therefrom to be sandwiched by Snapwell inserts, following which a layer of epidermal cells is formed over the piece of fibroblast layer that was sandwiched by the Snapwell inserts.

More specifically, operations such as the following have been required.
(1) A 6-well cell culture insert is seeded with a liquid mixture of collagen and fibroblasts of low cell density that will become the lower dermal layer. The number of fibroblast cells required is 0.9 × 10⁶.
(2) After the aforementioned liquid mixture of collagen and cells has become firm, a liquid mixture of collagen and fibroblasts of high cell density that will become the upper dermal layer is seeded thereabove. The number of fibroblast cells required is 3.7 × 10⁶.
(3) After these have become firm, and the dermis comprising the upper dermal layer and the lower dermal layer has formed, a ring-shaped cutter is used to cut out a piece of the dermis therefrom. In addition, the piece of the dermis that was cut out therefrom is captured within the lower container of a Snapwell insert.
(4) The upper container of the Snapwell insert is thereafter mated over the lower container of the Snapwell insert within which the piece of the dermis that was cut out therefrom was captured.
(5) A liquid suspension of epithelial cornified cells is seeded over the dermis that was captured within the Snapwell insert to form a layer of epithelial cornified cells.
(6) Culturing operations are carried out to obtain a tension-applied three-dimensional artificial skin.

However, when the foregoing procedure was used to attempt to produce a large number of tension-applied three-dimensional artificial skins, it became clear that there were a large number of problems, including those of time, cost, proficiency, and so forth.

In particular, with the foregoing procedure, a total of 4.6 × 10⁶ fibroblast cells per 6-well cell culture insert are used at operations (1) through (2). For this reason, because an extremely large number of fibroblast cells had been required so as to be able to provide a large number of tension-applied three-dimensional artificial skins, improvement was necessary, as the cost and time incurred during cell culture were enormous.

Furthermore, with the foregoing procedure, at operation (3), during the operation in which the piece of dermis is cut out therefrom and in which it is captured within the lower container of the Snapwell insert, this took much time, and proficiency had been required of the worker responsible for performing the work operation. In particular, because proficiency had been required of the responsible worker so as to permit a piece of dermis of prescribed shape to be cleanly cut out therefrom, this was a factor that resulted in increase in personnel cost.

Moreover, proficiency was also required at operation (4) where the upper container is mated to the lower container. In particular, ensuring that the height of the upper container was the same every time, i.e., causing the upper container to be mated to the lower container such that the distance between the bottom of the upper container and the bottom of the lower container was a constant distance, was highly difficult. Here, if the height and/or position of the upper container that is mated over the lower container is different for each Snapwell insert, this means that the three-dimensional artificial skins will be such that the tension applied thereto will be different for each Snapwell insert, causing product quality to be unstable despite passage through the subsequent operations (5) through (6); and furthermore, where the upper container that is mated thereto is inclined such that there is occurrence of places at which the height of the bottom of the upper container varies, this will cause occurrence of distortion in the tension at the three-dimensional artificial skin, as a result of which product quality will be unstable despite passage through the subsequent operations (5) through (6).

For this reason, the proficiency of the worker greatly affects the manufacturing yield of the tension-applied three-dimensional artificial skin that is ultimately obtained, and the success rate for manufacture of a tension-applied three-dimensional artificial skin according to the foregoing procedure has been on the order of 10% to 20% for a beginner, and has been on the order of 60% to 80% for an experienced professional.

Thus, because that involving the procedure up to this point has been such that handling during manufacturing operations has been complicated, production efficiency has not been high, proficiency has been demanded to obtain a stable tension-applied three-dimensional artificial skin, and formulation of standardized work procedures has been difficult, there has come to be demand for an easier and more highly efficient manufacturing method.

With the object of developing a container for easy, stable, and highly efficient production of a tension-applied three-dimensional artificial skin, and of developing a method for producing a tension-applied three-dimensional artificial skin, the present inventor(s) therefore through trial-and-error perfected the invention of the present application.

### MEANS FOR SOLVING PROBLEM

As a result of intensive and repeated research for the purpose of solving problems such as the problem of reduction in quality due to misalignment and the like at the various containers which can occur during the aforementioned conventional operation in which pieces of dermis are cut out from a 6-well insert and/or the operation in which an upper container is mated to a lower container, the present inventor(s) invented a culture insert container that is a novel culture insert container having a lower culture container having a bottom portion equipped with a porous membrane and a sidewall, and having an upper insertion fixture having a cylindrical sidewall that is inserted into said lower culture container, said cylindrical sidewall having a land arranged so as to protrude from a top end of the sidewall of the lower culture container at a location such as will cause a top surface of said porous membrane and a bottom end portion of said cylindrical sidewall which is inserted within the lower culture container to be maintained in a noncontacting state, and having a land arranged at an outer surface of the cylindrical sidewall so as to cause a constant distance to be maintained between an inner surface of the sidewall of the lower culture container and the outer surface of the cylindrical sidewall, and employed same to produce a tension-applied three-dimensional artificial skin.

A first means for solving the problems in accordance with the present invention is a culture insert container 1 having a lower culture container 3 having a bottom portion 33 equipped with a porous membrane 32 and a sidewall 31, and having an upper insertion fixture 2 having a cylindrical sidewall 21 that is inserted into said lower culture container, said cylindrical sidewall having a land 23 arranged so as to protrude from a top end of the sidewall of the lower culture container at a location such as will cause a top surface of said porous membrane and a bottom end portion of said cylindrical sidewall which is inserted within the lower culture container to be maintained in a noncontacting state, and having a land 25 arranged at an outer surface of the cylindrical sidewall so as to cause a constant distance to be maintained between an inner surface of the sidewall of the lower culture container and the outer surface of the cylindrical sidewall.

A second means for solving the problems in accordance with the present invention is the culture insert container 1 according to the first means characterized in that a surface of the bottom portion 22 of the cylindrical sidewall of the upper insertion fixture 2 has been subjected to surface roughening treatment.

A third means for solving the problems in accordance with the present invention is the culture insert container according to the first or second means characterized in that a land 23 for causing the cylindrical sidewall of the upper insertion fixture to be placed at a top end portion of the sidewall of the lower culture container has a wing, i.e., a wing-shaped portion 24. By causing this to be a land that has a wing-shaped portion, it will be possible to cause the culture insert container to be made such that placement with respect to a plate, multi-well plate, or other such culture container is facilitated.

A fourth means for solving the problems in accordance with the present invention is the culture insert container according to any one of the first through third means characterized in that the land arranged at the outer surface of the cylindrical sidewall of the upper insertion fixture is in a shape of a rib. By providing a plurality of lands arranged at the outer surface of the cylindrical sidewall of the upper insertion fixture that are rib-shaped, it will be possible to more stably maintain a constant gap with the sidewall without causing the upper insertion fixture to become inclined when it is inserted into the lower culture container.

A fifth means for solving the problems in accordance with the present invention is the culture insert container according to any one of the first through fourth means characterized in that it has a rotation stopper that prevents rotation of the upper insertion fixture, i.e., rotation stopping mechanism 26, 34 that engages with and secures the upper insertion fixture and the lower culture container. The rotation stopping mechanism might, for example, have convexity and/or concavity 34 serving as rotation stopper which is arranged toward the exterior at the sidewall of the lower culture container, and a catch portion 26 or the like provided at the upper insertion fixture which engages with said convexity and/or concavity. By providing a rotation stopping mechanism, it will be possible to suppress occurrence of situations in which an upper insertion fixture that is inserted into a lower culture container might otherwise inadvertently be made to rotate, making it possible to stably manufacture a tension-applied three-dimensional artificial skin.

A sixth means for solving the problems in accordance with the present invention is the culture insert container according to any one of the first through fifth means characterized in that the cylindrical sidewall is in a shape of a right circular cylinder.

In addition, a method for producing tension-applied three-dimensional artificial skin at a seventh means for solving the problems in accordance with the present invention employs the culture insert container 1 according to any one of the first through sixth means in accordance with the present invention having the lower culture container 3 having the bottom portion equipped with the porous membrane and the sidewall, and having the upper insertion fixture 2 having the cylindrical sidewall that is inserted into said lower culture container, said cylindrical sidewall having the land arranged so as to protrude from the top end of the sidewall of the lower culture container at the location such as will cause the top surface of said porous membrane and the bottom end portion of said cylindrical sidewall which is inserted within the lower culture container to be maintained in the noncontacting state, and having the land arranged at the outer surface of the cylindrical sidewall so as to cause the constant distance to be maintained between the inner surface of the sidewall of the lower culture container and the outer surface of the cylindrical sidewall, to manufacture a tension-applied three-dimensional artificial skin by means of operations such as are indicated below.

More specifically, a method for producing tension-applied three-dimensional artificial skin at the seventh means for solving the problems in accordance with the present invention employs the culture insert container according to any one of the first through sixth means in accordance with the present invention having the lower culture container having the bottom portion equipped with the porous membrane and the sidewall, and having the upper insertion fixture having the cylindrical sidewall that is inserted into said lower culture container, said cylindrical sidewall having the land arranged so as to protrude from the top end of the sidewall of the lower culture container at the location such as will cause the top surface of said porous membrane and the bottom end portion of said cylindrical sidewall which is inserted within the lower culture container to be maintained in the noncontacting state, and having the land arranged at the outer surface of the cylindrical sidewall so as to cause the constant distance to be maintained between the inner surface of the sidewall of the lower culture container and the outer surface of the cylindrical sidewall, and employs the lower culture container in dedicated fashion for formation of the dermis, and is accompanied by the following operations.
(1) A solution of collagen and fibroblasts of low cell density are directly seeded in the lower culture container. Where a 6-well culture insert container is used, the number of fibroblast cells required will be 1.0 × 10⁶.
(2) After the solution of collagen and fibroblasts of low cell density that was seeded has become firm, a solution of collagen and fibroblasts of high cell density is further seeded. Where a 6-well culture insert container is used, the number of cells required will be 1.8 × 10⁶.
(3) After the solution of collagen and fibroblasts of high cell density that was seeded has become firm and a dermis has formed, the upper insertion fixture is pressed against the lower culture container until it stops and is secured thereto.
(4) A liquid suspension of epithelial cornified cells is seeded over the dermis that was formed, and a layer of epithelial cornified cells is formed.
(5) Culturing operations are carried out to obtain the tension-applied three-dimensional artificial skin.

### BENEFIT OF INVENTION

Culture insert containers at means in accordance with the present invention, and methods for producing tension-applied three-dimensional artificial skins employing same at means in accordance with the present invention, make it possible, by replacing culture insert container(s) used at operation(s) for manufacture of tension-applied three-dimensional artificial skin(s) with culture insert container(s) at means in accordance with the present invention, to eliminate the operation in which piece(s) are cut out of the dermis which is formed. As a result, whereas for a 6-well culture insert container the number of fibroblast cells required to manufacture tension-applied three-dimensional artificial skin had conventionally been 4.6 × 10⁶, means in accordance with the present invention make it possible to reduce this to 2.8 × 10⁶. Moreover, due to the fact that with culture insert containers at means in accordance with the present invention, and methods for producing tension-applied three-dimensional artificial skins employing same at means in accordance with the present invention, there is no operation in which something must be mated to the lower container of the Snapwell insert, an experienced professional can achieve a production efficiency which is close to 100%, dramatically reducing the time required. Reductions in time and improvements in efficiency also make it possible to increase the number of artificial skins that can be manufactured at any given time, and the time it takes to become familiar with the technology is dramatically reduced thereby. Furthermore, whereas, with a conventional culture insert container and method for producing a tension-applied three-dimensional artificial skin employing same, adjustment of height when causing the upper container to be mated to the lower container of the Snapwell insert was technically problematic, because, with the culture insert container of a means in accordance with the present invention, the height with which the upper insertion fixture is made to mate to the lower culture container can be set so as to be constant, and height can be accurately controlled every time, it is possible to dramatically improve work efficiency and the yield with which the tension-applied three-dimensional artificial skin is manufactured. The present invention makes it possible improve the number of skins that can be manufactured per unit time by 2× or more, success rates therewith being 60% to 80% even for a complete beginner, with this success rate further increasing after a few tries and making it possible to achieve a production efficiency which is close to that of an experienced professional. Thus, culture insert containers at means in accordance with the present invention, and methods for producing tension-applied three-dimensional artificial skins employing same at means in accordance with the present invention, by improving yield and eliminating various operations requiring proficiency, are able to dramatically increase production efficiency, and permit reduction in the amount of fibroblast cells that are used, without requiring an operation in which a piece is cut out therefrom.

### BRIEF DESCRIPTION OF DRAWINGS

[FIG. 1] Shows upper insertion fixture in culture insert container at a means in accordance with the present invention. (a) is plan, (b) is front, and (c) shows overview from bottom.
[FIG. 2] Shows (a) plan view, (b) rear view, (c) left side view, (d) right side view, (e) plan view, (f) bottom view, and (g) drawings depicting rear, left side, and plan views of upper insertion fixture in culture insert container at a means in accordance with the present invention.
[FIG. 3] Shows lower culture container in culture insert container at a means in accordance with the present invention. (a) is plan, (b) is front, and (c) shows overview from bottom.
[FIG. 4] Shows (a) plan view, (b) rear view, (c) left side view, (d) right side view, (e) plan view, (f) bottom view, and (g) drawings depicting rear, left side, and plan views of lower culture container in culture insert container at a means in accordance with the present invention.
[FIG. 5] Shows upper insertion fixture and lower culture container, these being shown in their engaged state, in a culture insert container at a means in accordance with the present invention. (a) is plan, (b) is front, and (c) shows overview from bottom.
[FIG. 6] Shows (a) plan view, (b) rear view, (c) left side view, (d) right side view, (e) plan view, (f) bottom view, and (g) drawings depicting rear, left side, and plan views of upper insertion fixture and lower culture container, these being shown in their engaged state, in a culture insert container at a means in accordance with the present invention.
[FIG. 7] Shows upper insertion fixture and lower culture container, these being shown in their engaged state, in a culture insert container at a means in accordance with the present invention.
[FIG. 8] Shows operations for manufacture of a conventional tension-applied three-dimensional artificial skin.
[FIG. 9] Shows operations for manufacture of a tension-applied three-dimensional artificial skin at a means in accordance with the present invention.
[FIG. 10] Shows results of testing in which dermal structure was confirmed by H&E staining.

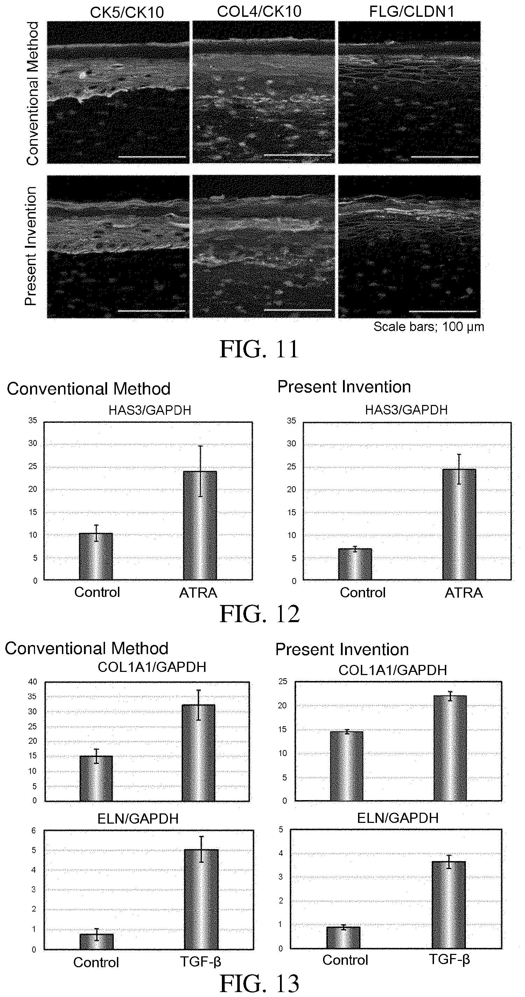

[FIG. 11] Shows results of testing in which epidermal structure was confirmed by immunostaining.
[FIG. 12] Shows results of testing in which the amount of expression of HAS3 mRNA 6 hours after addition of ATRA was measured.
[FIG. 13] Shows results of testing in which expression of collagen 1A1 and elastin 48 hours after addition of TGF-β was measured.

### EMBODIMENTS FOR CARRYING OUT INVENTION

Below, embodiments of the present invention are described in concrete terms with reference as appropriate to the drawings and descriptions of working examples. Note, however, that said descriptions should not be interpreted in a manner that would limit the form taken by the container for producing tension-applied three-dimensional artificial skin, and production method therefor, of the present invention.

So long as the effects of the present invention are provided thereby, the present invention is such that embodiments combining any of the various constitutions described at the foregoing means in accordance with the present invention are also included within the technical scope of the present invention.

Below, although the present invention is described in more concrete terms by way of working examples, the present invention should not be understood to be limited in any manner by these working examples, as a large number of variations within the purview of the technical idea of the present invention will be possible for a person having ordinary knowledge in the pertinent field.

### WORKING EXAMPLES

### Working Examples

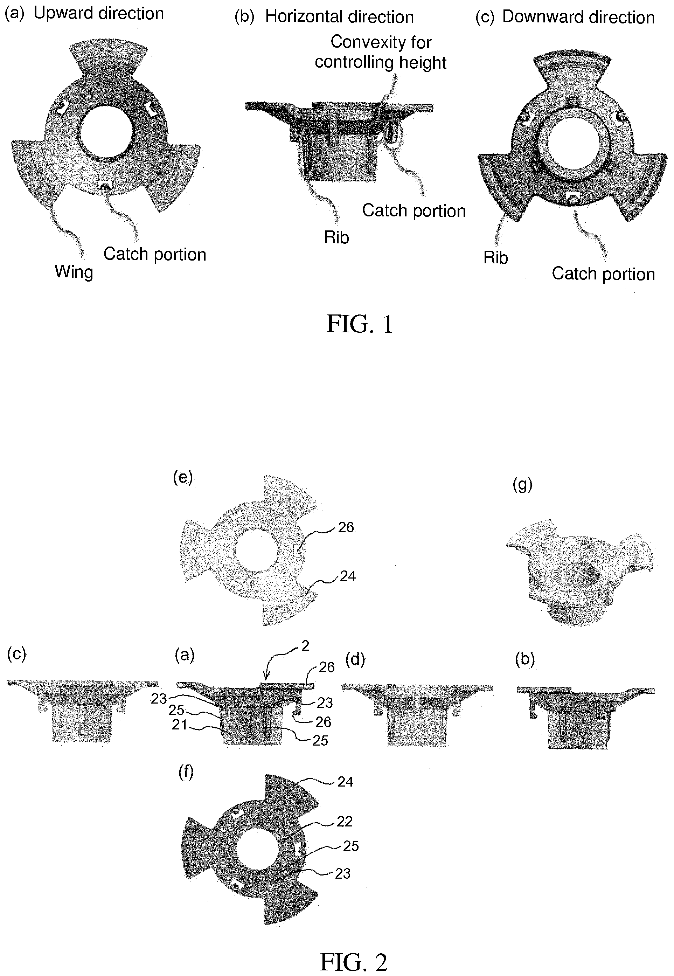

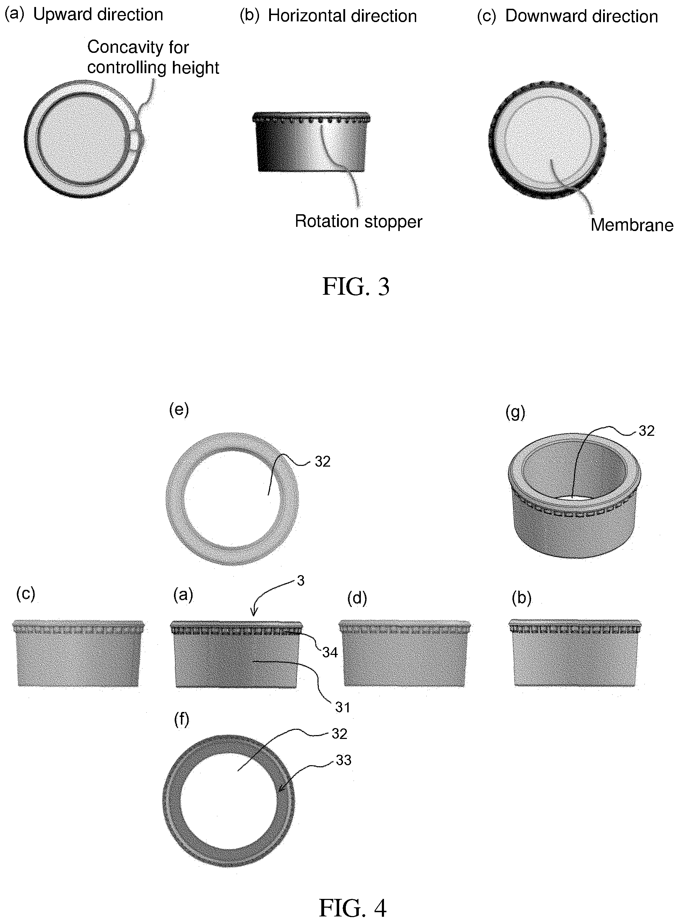

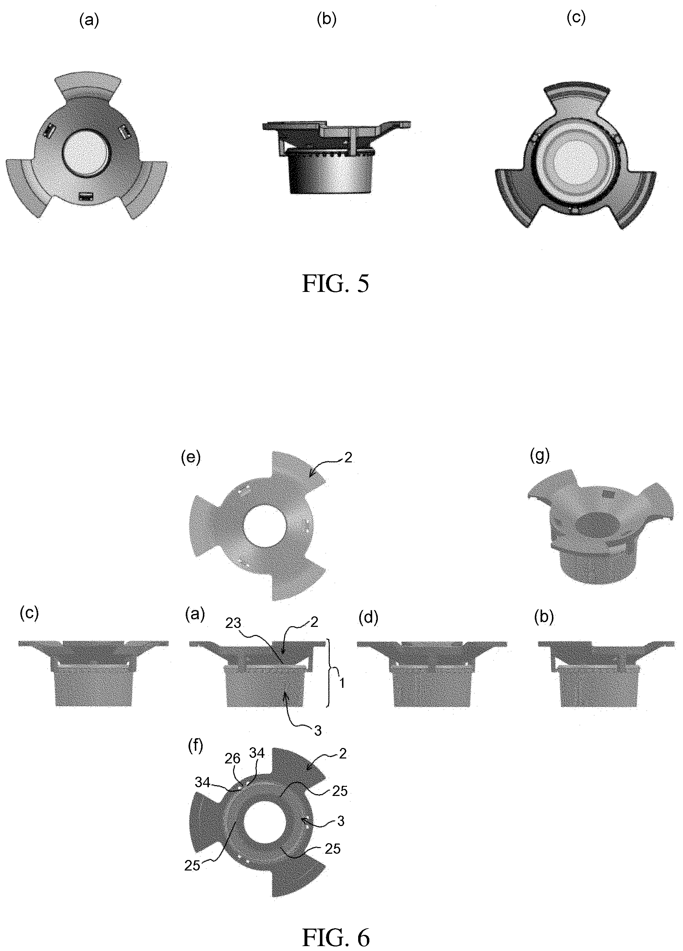

As a culture insert container at a means in accordance with the present invention, polystyrene and a porous membrane were used to manufacture a culture insert container having a lower culture container having a bottom portion equipped with a porous membrane and a sidewall with structure shown in FIGS. 1 through 7, and having an upper insertion fixture having a cylindrical sidewall that is inserted into said lower culture container, said cylindrical sidewall having a land arranged so as to protrude from a top end of the sidewall of the lower culture container at a location such as will cause a top surface of said porous membrane and a bottom end portion of said cylindrical sidewall which is inserted within the lower culture container to be maintained in a noncontacting state, and having a land arranged at an outer surface of the cylindrical sidewall so as to cause a constant distance to be maintained between an inner surface of the sidewall of the lower culture container and the outer surface of the cylindrical sidewall. Note that culture insert containers at means in accordance with the present invention might, for example, be manufactured using polystyrene, polyethylene, polyethylene terephthalate, polypropylene, glass, metal, ceramic, and/or other such material(s) capable of being used for cell culture.
1) Tension-applied three-dimensional artificial skins manufactured in accordance with methods for producing tension-applied three-dimensional artificial skins employing culture insert containers at means in accordance with the present invention will be evaluated by comparison with tension-applied three-dimensional artificial skins produced by conventional manufacturing methods.

### (1) Cell Culture

Cells used were dermal fibroblasts (manufactured by Kurabo) and epithelial cornified cells (manufactured by Kurabo). The dermal fibroblasts were cells recovered after being cultured according to the Kurabo instruction manual until 90% confluency was reached. The epithelial cornified cells were cells recovered after being cultured according to the Kurabo instruction manual until 70% to 80% confluency was reached.

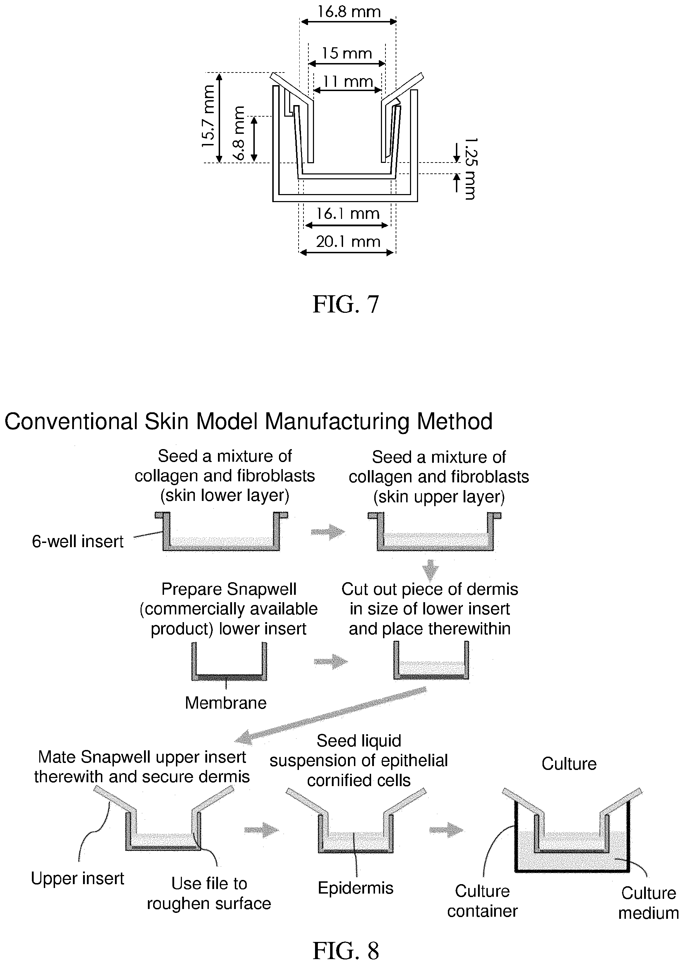

### (2) Manufacture of Artificial Skin in Accordance with Conventional Art (Also See FIG. 8).

Dermal fibroblasts and Atelocollagen gel (manufactured by Koken) adjusted to 4 mg/mL were mixed to prepare that which was used for the lower layer (0.9 × 10⁶ cells/1.8 mL/well) and that which was used for the upper layer (3.7 × 10⁶ cells/0.9 mL/well).

The gel for the lower layer was added to a 6-well cell culture insert, and this was allowed to stand for 30 minutes in 5% CO₂ and 12.5% CO₂ incubators at 37° C. The gel for the upper layer was added over the solidified lower dermal layer, and this was allowed to stand for 30 minutes in 5% CO₂ and 12.5% CO₂ incubators at 37° C.

A ring-shaped cutter that was 11 mm in diameter was used to cut out a piece of dermis therefrom, this was placed in the lower container of a Snapwell insert in which 50 µL of Atelocollagen gel had been spread, and this was adjusted so as to cause the artificial skin to be horizontal.

200 µL of 0.5% Matrigel solution was added thereto, and this was allowed to stand for 1 hour in 5% CO₂ and 12.5% CO₂ incubators at 37° C. The upper container of the Snapwell insert was mated and secured to the lower container. The epithelial cornified cells were suspended in Humedia-KG2 (manufactured by Kurabo), and this was seeded at 1.1 × 10⁶ cells/0.5 mL/well.

This was placed in a 6-well plate, 7 mL of artificial skin culture medium (Dulbecco's Modified Eagle's Medium containing 1 µM hydrocortisone, 5 µg/mL insulin, 1 mM L-ascorbic acid, 10 mg/mL b-FGF, and 10% fetal bovine serum) was added thereto, and culture was initiated in 5% CO₂ and 12.5% CO₂ incubators at 37° C. At the epidermal side, medium was replaced every day for the first 3 days of culture. At the dermal side, medium was replaced every other day. On the 4th day of culture, the medium on the epidermal side was completely removed, and air layer culture was initiated. On the 6th day of culture and thereafter, culture was carried out in an incubator at 5% CO₂ and 37° C.

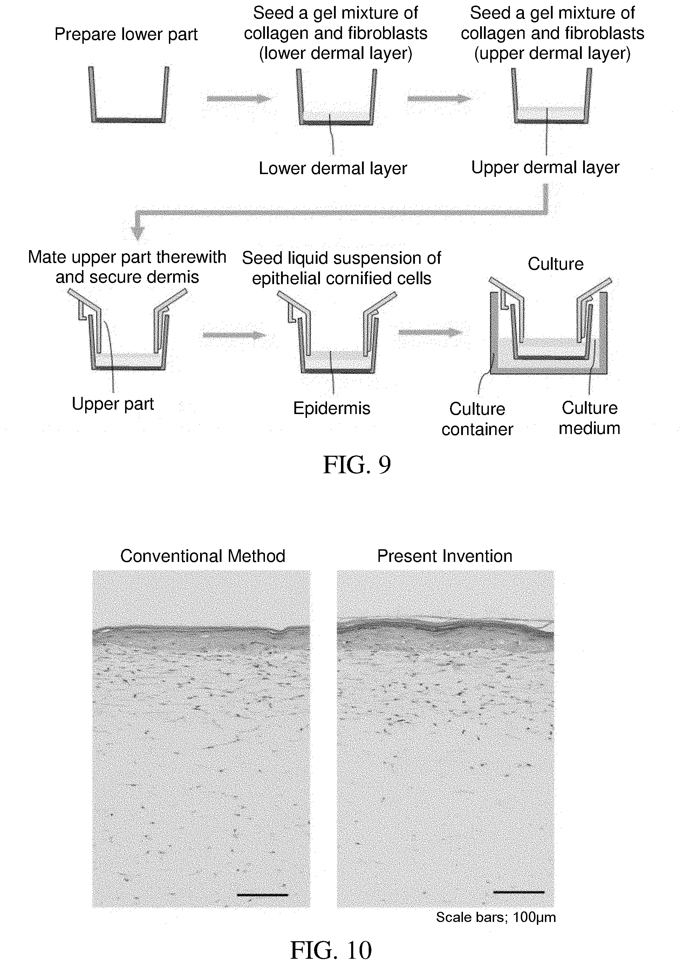

### (3) Manufacturing Method at Means in Accordance with Present Invention (Also See FIG. 9).

Dermal fibroblasts and Atelocollagen gel (manufactured by Koken) adjusted to 4 mg/mL were mixed to prepare that which was used for the lower layer (1.0 × 10⁶ cells/0.9 mL/well) and that which was used for the upper layer (1.8 × 10⁶ cells/0.3 mL/well).

The gel for the lower layer was added to the lower culture container of the present invention, and this was allowed to stand for 30 minutes in 5% CO₂ and 12.5% CO₂ incubators at 37° C.

The gel for the upper layer was added over the solidified lower dermal layer, and this was allowed to stand for 30 minutes in 5% CO₂ and 12.5% CO₂ incubators at 37° C.

The upper insertion fixture was placed over the lower culture container, a load was slowly placed thereon, this being pressed thereonto until it stopped and was secured thereto.

200 µL of 0.5% Matrigel solution was added thereto, and this was allowed to stand for 1 hour in 5% CO₂ and 12.5% CO₂ incubators at 37° C. The epithelial cornified cells were suspended in Humedia-KG2 (manufactured by Kurabo), and this was seeded at 1.8 × 10⁶ cells/0.7 mL/well.

This was placed in a 6-well plate, 7 mL of artificial skin culture medium (Dulbecco's Modified Eagle's Medium containing 1 µM hydrocortisone, 5 µg/mL insulin, 1 mM L-ascorbic acid, 10 mg/mL b-FGF, and 10% fetal bovine serum) was added thereto, and culture was initiated in 5% CO₂ and 12.5% CO₂ incubators at 37° C. At the epidermal side, medium was replaced every day for the first 3 days of culture. At the dermal side, medium was replaced every other day. On the 4th day of culture, the medium on the epidermal side was completely removed, and air layer culture was initiated. On the 6th day of culture and thereafter, culture was carried out in an incubator at 5% CO₂ and 37° C.

2) Histological Comparison of Tension-Applied Three-Dimensional Artificial Skin Model Obtained by Means in Accordance with Present Invention and Tension-Applied Artificial Skin Model Manufactured in Accordance with Conventional Method

On the 11th day of culture, each artificial skin was washed twice with 7 mL of PBS (-), and was thereafter fixed overnight at 4° C in 7 mL of 4% PFA/PB. Following paraffin embedding, these were sectioned, and hematoxylin-eosin (HE) staining and immunohistochemical staining were carried out. TABLE 1 indicates the staining conditions and antibodies that were used for immunohistochemical staining.

**TABLE 1**

| Abbreviation | Name of Antibody | Manufacturer | cat # | Dilution Ratio |
|---|---|---|---|---|
| CK10 | Anti-Cytokeratin 10 antibody | Abcam | ab9026 | 1/200 |
| CK5 | Anti-Cytokeratin 5 antibody | Abcam | ab52635 | 1/200 |
| CLDN1 | Claudin 1 Antibody | Thermo Fisher Scientific | 71-7800 | 1/100 |
| COL4 | Anti-Collagen IV antibody | Abcam | ab6586 | 1/500 |
| FLG | Anti-Filaggrin antibody [SPM181] | Abcam | ab 17808 | 1/50 |

Regardless of the manufacturing method employed, all artificial skins that were prepared were such that two dermal layers were formed, the fibroblasts being of differing cell densities therein; and such that tension had been applied, as the cells were elongated laterally. Furthermore, CK5-positive basal cells were arrayed in well-ordered rows, COL4 being expressed as if to underlie the basal layer. Prickle cell layer Claudin and granule cell layer Filaggrin were moreover formed in layered fashion. These results indicated that, in similar fashion as with the conventional method, the tension-applied three-dimensional artificial skin obtained by means in accordance with the present invention was such that there was formation of an epidermis with differentiation into four layers, in similar fashion as with the tension-applied artificial skin manufactured in accordance with the conventional method.

3) Comparison of Gene Expression in Tension-Applied Three-Dimensional Artificial Skin Model Obtained in Accordance with Present Invention and in Tension-Applied Artificial Skin Manufactured in Accordance with Conventional Method

### a) Expression of HAS3 Gene

The artificial skin was such that, on the 6th day of culture, this was replaced with a medium for assay use (Dulbecco's Modified Eagle's Medium containing 1 µM hydrocortisone, 5 µg/mL insulin, and 10% fetal bovine serum). On the 8th day of culture, gauze that had been impregnated with 0.2% HPC solution or 10 µM ATRA solution was placed on the surface of the epidermis, and this was cultured for 1 hour in a 5% CO₂ incubator at 37° C. After 1 hour, the gauze was removed, and this was further cultured for 5 hours in a 5% CO₂ incubator at 37° C. Following culture of the artificial skin for 6 hours, the artificial skin was removed from the container in such fashion as to not cause the epidermal tissue to be delaminated therefrom, and a razor was used to cut this in half. After using RNT solution from an RNeasy Plus Mini Kit (manufactured by Qiagen) to dissolve tissue by pulverizing with beads, phenol chloroform was used to remove protein therefrom, following which column purification was carried out as directed by the RNeasy Plus Mini Kit protocol to obtain total RNA.

SuperScript VILO Master Mix (manufactured by Invitrogen) was used as directed by the attached manual to carry out synthesis of cDNA from the purified total RNA. The synthesized cDNA was mixed with SYBR Premix Ex Taq (manufactured by Takara) and primer mix (a mixture of forward and reverse primers), 12.5 µL of reaction solution was prepared in each well of a 96-well plate for PCR use, and a QuantStudio 12K Flex (Applied Biosystems) was used to carry out real-time PCR. The real-time PCR reaction was such that 40 cycles comprising 95° C for 30 seconds for initial denaturation, and 95° C for 5 seconds and 60° C for 31 seconds for the PCR reaction, were carried out. TABLE 2 shows the sequences of the primers (manufactured by Thermo Fisher Science) for amplifying the genes that were used.

**TABLE 2**

| Gene | Forward | Reverse |
|---|---|---|
| GAPDH | TCTGACTTCAACAGCGACAC | CCCTGTTGCTGTAGCCAAATTC |
| HAS3 | CGCAGCAACTTCCATGAGG | AGTCGCACACCTGGATGTAGT |
| COL1A1 | AGGAAGGCCAAGTCGAGG | CCGGGGCAGTTCTTGGTC |
| ELN | TCCTGCTGTCCATCCTCCAC | AAAAGACTCCTCCAGGAACTCC |

Analysis of hyaluronan synthase (HAS3) gene expression indicated that gene expression in the artificial skin of the present invention increased by 3× or more as compared with the situation when unstimulated, indicating that it had a reactivity that was equivalent to or better than that of the conventional method (FIG. 9).

### b) Collagen 1A1 and Elastin Expression

At the artificial skin, on the 6th day of culture, this was replaced with a medium for assay use (Dulbecco's Modified Eagle's Medium containing 1 µM hydrocortisone, 5 µg/mL insulin, and 10% fetal bovine serum). On the 8th day of culture, this was replaced with assay medium that contained or that did not contain 10 ng/mL TGF-β. Medium was replaced 24 hours thereafter, and culturing was carried out for 48 hours. After 48 hours, RNA was extracted from the artificial skin in similar fashion as at 3-a), following which cDNA was synthesized, and real-time PCR was performed. The sequences of the primers (manufactured by Thermo Fisher Science) for amplifying the genes that were used are those indicated at TABLE 2.

Analysis of collagen 1A1 (COL1A1) and elastin (ELN) gene expression indicated that, as compared with the situation when unstimulated, artificial skins prepared using means in accordance with the present invention were such that gene expression increased by 1.5× or more for COL1A1, and gene expression increased by 3 × or more for elastin, indicating that these had reactivities equivalent to those of the conventional method (FIG. 10).

### INDUSTRIAL UTILITY

Means in accordance with the present invention make it possible, by improving yield and eliminating various operations requiring proficiency, to dramatically increase production efficiency, and permit reduction in the amount of fibroblast cells that are used, without requiring an operation in which a piece is cut out therefrom. In addition, because it makes it possible to eliminate the operation in which a piece is cut out from the dermis which is formed, taking the case of a 6-well culture insert container, this means that the number of fibroblast cells necessary to manufacture a tension-applied three-dimensional artificial skin can be reduced to 2.8 × 10⁶ cells from the 4.6 × 10⁶ cells that had been necessary conventionally. Furthermore, because with means in accordance with the present invention there is no operation in which something must be mated to the lower culture container of the Snapwell insert, an experienced professional can achieve a production efficiency which is close to 100%, dramatically reducing the time required. Furthermore, whereas, with a conventional culture insert container and method for producing a tension-applied three-dimensional artificial skin employing same, adjustment of height when causing the upper container to be mated to the lower container of the Snapwell insert was technically problematic, because, with the culture insert container of a means in accordance with the present invention, the height with which the upper insertion fixture is made to mate to the lower culture container can be set so as to be constant by means of a land that causes a cylindrical sidewall to be placed at a sidewall of the lower culture container, and the height at which the upper insertion fixture is inserted can be accurately controlled so as to be a constant value every time even when done by someone who is not an experienced professional, work efficiency, and the yield with which the tension-applied three-dimensional artificial skin is manufactured, are dramatically improved, and the number of tension-applied three-dimensional artificial skins that can be manufactured per unit time is improved by 2× or more as compared with that which was possible conventionally. Reductions in time and improvements in efficiency furthermore make it possible to increase the number of artificial skins that can be manufactured at any given time, and the time it takes to become familiar with the technology is dramatically reduced. Means in accordance with the present invention make it possible to achieve success rates as high as 60% to 80% to manufacture tension-applied three-dimensional artificial skins even when this is performed by a complete beginner, and since after a few tries this success rate will increase and make it possible to achieve a production efficiency which is close to that of an experienced professional, dramatic reduction in manufacturing cost, including personnel cost, is made possible thereby.

### EXPLANATION OF REFERENCE NUMERALS

- 1: Culture insert container
- 2: Upper insertion fixture
- 3: Lower culture container
- 21: Cylindrical sidewall
- 22: Bottom end portion of cylindrical sidewall
- 23: Land arranged so as to protrude from top end of sidewall of lower culture container at location such as will cause top surface of said porous membrane and bottom end portion of said cylindrical sidewall which is inserted within lower culture container to be maintained in noncontacting state
- 24: Wing-shaped portion
- 25: Land arranged at outer surface of cylindrical sidewall
- 26: Rotation stopping mechanism
- 31: Sidewall
- 32: Porous membrane
- 33: Bottom portion
- 34: Rotation stopping mechanism

## Claims

1. A culture insert container having a lower culture container having a bottom portion equipped with a porous membrane and a sidewall, and having an upper insertion fixture having a cylindrical sidewall that is inserted into said lower culture container, said cylindrical sidewall having a land arranged so as to protrude from a top end of the sidewall of the lower culture container at a location such as will cause a top surface of said porous membrane and a bottom end portion of said cylindrical sidewall which is inserted within the lower culture container to be maintained in a noncontacting state, and having a land arranged at an outer surface of the cylindrical sidewall so as to cause a constant distance to be maintained between an inner surface of the sidewall of the lower culture container and the outer surface of the cylindrical sidewall.

2. The culture insert container according to claim 1 **characterized in that** a surface of the bottom portion of the cylindrical sidewall of the upper insertion fixture has been subjected to surface roughening treatment.

3. The culture insert container according to claim 1 **characterized in that** a land for causing the cylindrical sidewall of the upper insertion fixture to be placed at a top end portion of the sidewall of the lower culture container has a wing-shaped portion.

4. The culture insert container according to claim 1 **characterized in that** the land arranged at the outer surface of the cylindrical sidewall of the upper insertion fixture is in a shape of a rib.

5. The culture insert container according to claim 1 **characterized in that** it has a rotation stopping mechanism that engages with and secures the upper insertion fixture and the lower culture container.

6. The culture insert container according to claim 1 **characterized in that** the cylindrical sidewall is in a shape of a right circular cylinder.

7. A method for producing tension-applied three-dimensional artificial skin that employs the culture insert container according to any one of claims 1 through 6 having the lower culture container having the bottom portion equipped with the porous membrane and the sidewall, and having the upper insertion fixture having the cylindrical sidewall that is inserted into said lower culture container, said cylindrical sidewall having the land arranged so as to protrude from the top end of the sidewall of the lower culture container at the location such as will cause the top surface of said porous membrane and the bottom end portion of said cylindrical sidewall which is inserted within the lower culture container to be maintained in the noncontacting state, and having the land arranged at the outer surface of the cylindrical sidewall so as to cause the constant distance to be maintained between the inner surface of the sidewall of the lower culture container and the outer surface of the cylindrical sidewall, and that is **characterized in that** it has the operations indicated below.
(1) A first operation in which a solution of collagen and fibroblasts of low cell density are directly seeded in the lower culture container.
(2) A second operation in which, after the solution of collagen and fibroblasts of low cell density that was seeded has become firm, a solution of collagen and fibroblasts of high cell density is further seeded.
(3) A third operation in which, after the solution of collagen and fibroblasts of high cell density that was seeded has become firm and a dermis has formed, the upper insertion fixture is pressed against the lower culture container until it stops and is secured thereto.
(4) A fourth operation in which a liquid suspension of epithelial cornified cells is seeded over the dermis that was formed, and a layer of epithelial cornified cells is formed.
(5) A fifth operation in which culturing operations are carried out to obtain the tension-applied three-dimensional artificial skin.
